# EUROPEAN PATENT APPLICATION

(11) **EP 4 740 977 A1**
(43) Date of publication of application: **13.05.2026**
(21) Application number: 25214724.4
(22) Date of filing: 10.11.2025
(51) Int. Cl.: A61M 3/02

(54) **SINGLE-USE BELLOWS MICROENEMA**

(30) Priority: 11.11.2024 IT 202400025323
(71) Applicant: INGE S.p.A., 20024 Garbagnate Milanese (IT)
(72) Inventor: NOBBIO, Alessio, 20145 Milano (MI) (IT)
(74) Representative: Di Gennaro, Sergio

(57) **Abstract**

A disposable microenema comprising a container (2), made of a flexible plastic material, surmounted by a neck (3) on which a cannula is inserted for dispensing a liquid solution contained in the container, said container comprising a bellows portion comprising a plurality of accordion-like folds (21) which, when pressed one on top of the other, reduce the internal volume of the container, forcing the liquid contained therein to flow out of the neck (3) through said cannula. The bottom of the container has a concave portion (22) designed to facilitate the manual pressure that must be applied in order to crush the bellows portion which has a thickness of the flexible plastic material lower than the thickness of the other parts of the container.

## Description

The present invention refers to a disposable bellows microenema. A microenema is a disposable device used to perform a clyster, generally in a home environment. Enemas and microenemas are at the forefront as fast-acting support in emergency cases of constipation, but also in order to support medical and pharmacological therapies. Said bellows microenemas generally comprise a tubular or truncated-conical cannula adapted to dispense a liquid solution, and a reservoir in which said solution is contained.

The reservoir of the microenema is usually a bellows container, made of a flexible plastic material which, when pressed from its bottom, is crushed and conveys the solution contained therein into the cannula.

The effectiveness of the microenema substantially depends on how completely, quickly, and easily the liquid solution can be dispensed. Therefore, the effectiveness substantially depends on the features of the reservoir and on its compressibility. Indeed, to avoid possible breakage or damage to the reservoir, its bottom is often reinforced, making its thickness greater than that of the other compressible parts. This can lead to difficulty in compressing the reservoir itself, which mainly occurs by pressing the microenema from its bottom.

The technical problem associated with the present invention is how to improve the compressibility of the reservoir of a microenema to effectively ensure the liquid substance therein to be dispensed through the cannula.

An aspect of the present invention relates to a disposable bellows microenema having the features of the attached claim 1.

Further features of the invention are comprised in the dependent claims.

The features and advantages of the disposable bellows microenema according to the present invention will become more apparent from the following exemplary and nonlimiting description, referred to the enclosed schematic drawings in which:
- Figure 1 illustrates a perspective view of the microenema in which only the bellows is shown, without the cannula, according to a first embodiment of the present invention;
- Figure 2 illustrates a side view of the microenema of Figure 1;
- Figure 3 illustrates a perspective view of the microenema in which only the bellows is shown, without the cannula, according to a second embodiment of the present invention;
- Figure 4 illustrates a side view of the microenema of Figure 3.

With reference to the cited figures, the microenema according to the present invention comprises a container 2, made of a flexible plastic material, surmounted by a neck 3 on which a cannula (not shown) is inserted for dispensing the liquid solution contained in the container 2. The container comprises a bellows portion comprising a plurality of accordion-like folds 21 which, when pressed one on top of the other, reduce the internal volume of the container, forcing the liquid contained therein to flow out through neck 3.

In said neck 3, there is an inlet portion 31 having a truncated-conical shape and a collar 32 on which the spout abuts.

The bottom of the container has a concave portion 22 designed to facilitate the manual pressure that must be applied to crush the bellows portion. To this end, said concave portion has a thickness of the flexible plastic material lower than the thickness of the other parts of the container. For example, in said concave portion, the thickness is between 5-10% lower than the thickness of the bellows portion.

Furthermore, compared to commercially available microenemas, the ratio between the height H of the bellows portion and the width L of the same portion is significantly lower. Whereas in traditional microenemas such ratio is between 1.5 and 2.5, in the present invention such ratio is between 0.8 and 1.3.

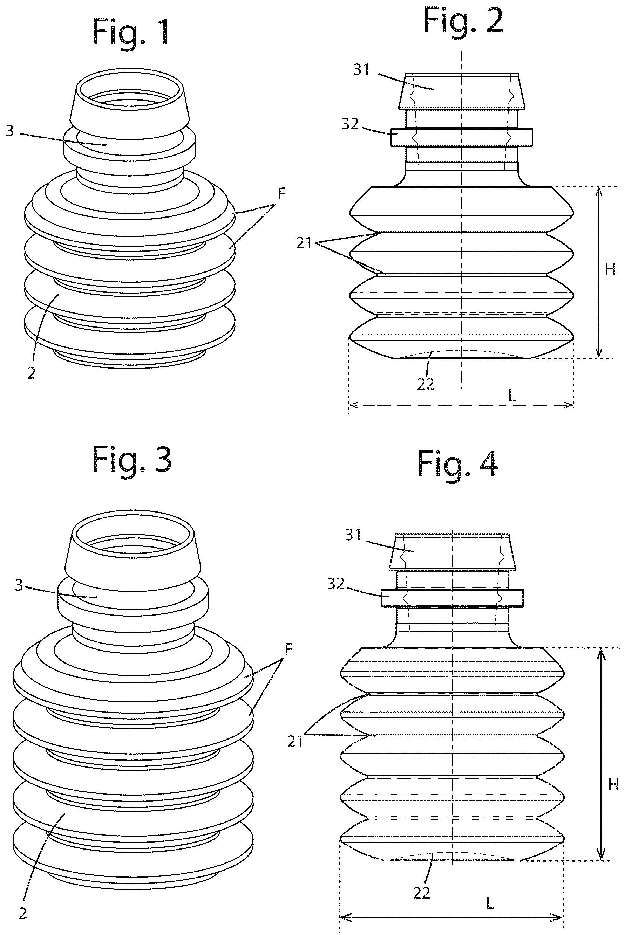

In the example illustrated in Figures 1 and 2, wherein the bellows comprises four flaps F, such ratio is approximately 0.8, while in the example illustrated in Figures 3 and 4, wherein the bellows comprises five flaps F, such ratio is approximately 1.1.

In practice, such dimensional ratios allow better compressibility of the container with the same amount of contained liquid.

## Claims

1. Disposable microenema comprising a container (2), made of a flexible plastic material surmounted by a neck (3) on which a cannula is inserted for dispensing a liquid solution contained in the container, said container comprising a bellows portion comprising a plurality of accordion-like folds (21) which when pressed one on top of the other reduce the internal volume of the container forcing the liquid contained therein to flow out of the neck (3) through said cannula,
**characterised in that** the bottom of the container has a concave portion (22) designed to facilitate the manual pressure that must be applied to crush the bellows portion which has a thickness of the flexible plastic material lower than the thickness of the other parts of the container.

2. Disposable microenema according to claim 1, wherein said concave portion (22) has a thickness between 5-10% lower than the thickness of the bellows portion.

3. Disposable microenema according to claim 1, wherein the ratio between the height (H) of the bellows portion and the width (L) of the same portion is between 0.8 and 1.3.

4. Disposable microenema according to claim 3, wherein the bellows portion comprises four flaps (F) and such ratio between the height (H) of the bellows portion and the width (L) of the same portion is approximately 0.8.

5. Disposable microenema according to claim 3, wherein the bellows portion comprises five flaps (F) and such ratio between the height (H) of the bellows portion and the width (L) of the same portion is approximately 1.1.
